# EUROPÄISCHE PATENTSCHRIFT

(11) **EP 1 794 113 B1**
(45) Veröffentlichungstag und Bekanntmachung des Hinweises auf die Patenterteilung: **09.07.2008**
(21) Anmeldenummer: 05769806.0
(22) Anmeldetag: 04.08.2005
(51) Int. Cl.: C07C 213/10, C07C 215/08

(54) **VERFAHREN ZUR HERSTELLUNG VON TRIETHANOLAMIN**
TRIETHANOLAMIN PRODUCTION METHOD
PROCEDE DE PRODUCTION DE TRIETHANOLAMINE

(30) Priorität: 31.08.2004 DE 102004042453
(43) Veröffentlichungstag der Anmeldung: 13.06.2007
(73) Patentinhaber: BASF SE, 67056 Ludwigshafen (DE)
(72) Erfinder: HAESE, Frank, 24855 Bollingstedt (DE); MELDER, Johann-Peter, 67459 Böhl-Iggelheim (DE); DOSTALEK, Roman, 67271 Neuleiningen (DE); JULIUS, Manfred, 67117 Limburgerhof (DE)
(86) Internationale Anmeldenummer: PCT/EP2005/008441
(87) Internationale Veröffentlichungsnummer: WO 2006/024358

(56) Entgegenhaltungen:
- EP-A- 0 004 015
- US-A- 5 693 866
- US-B1- 6 388 137
- US-B2- 6 323 371

## Beschreibung

Die vorliegende Erfindung betrifft ein Verfahren zur Herstellung von Triethanolamin und Triethanolamin enthaltend phosphorige und/oder unterphosphorige Säure und bestimmte basische Verbindungen.

Wichtige Einsatzgebiete von Triethanolamin (TEOA) oder dessen Folgeprodukten sind beispielsweise Seifen, Waschmittel und Shampoos in der kosmetischen Industrie oder auch Dispergiermittel und Emulgiermittel.

Für diese und andere Einsatzgebiete ist wasserklares, farbloses Triethanolamin mit einer möglichst geringen Verfärbung, z. B. gemessen als APHA- oder Gardner-Farbzahl, das diese Eigenschaften auch über längere Lagerzeiten (von z. B. 6, 12 oder mehr Monaten) beibehält, erwünscht.

Ein bekanntes Problem ist, dass ein nach einer fraktionierenden Destillation eines Triethanolamin-Rohprodukts, das z. B. durch Umsetzung von Ammoniak mit Ethylenoxid gewonnen wurde, erhaltenes reines TEOA eine gelbliche bis bräunliche oder rosa Verfärbung aufweist (Farbzahl z. B. ca. 10 bis 500 APHA nach DIN ISO 6271 (= Hazen)). Diese Verfärbung tritt besonders in Prozessen auf, in denen hohe Temperaturen durchlaufen werden.
Bei einer Lagerung des Alkanolamins, auch im geschlossenen Gebinde und unter Lichtausschluss, wird diese Verfärbung noch weiter verstärkt. (Siehe z. B.: T.I. MacMillan, Ethylene Oxide Derivatives, Report No. 193, Kapitel 6, Seiten 6-5 und 6-9 bis 6-13, 1991, SRI International, Menlo Park, California 94025;
G.G. Smirnova et al., J. of Applied Chemistry of the USSR 61, S. 1508-9 (1988), und Chemical & Engineering News 1996, Sept. 16, Seite 42, mittlere Spalte).

In der Literatur sind verschiedene Verfahren zur Herstellung von Triethanolamin mit verbesserter Farbqualität beschrieben.

EP-A-36 152 und EP-A-4015 (beide BASF AG) erläutern den Einfluss der in Verfahren zur Herstellung von Alkanolaminen eingesetzten Werkstoffe auf die farbliche Qualität der Verfahrensprodukte und empfehlen nickelfreie bzw. nickelarme Stähle.

US-A-3,207,790 (Dow Chemical Company) beschreibt ein Verfahren zur Verbesserung der Farbqualität von Alkanolaminen durch Zugabe eines Borhydrids eines Alkalimetalls.

EP-A-1 081 130 (BASF AG) betrifft ein Verfahren zur Herstellung von Alkanolaminen mit verbesserter Farbqualität durch Behandlung des Alkanolamins mit Wasserstoff in Gegenwart eines Hydrierkatalysators.

EP-A-4015 (BASF AG) beschreibt, dass Mono-, Di- und Triethanolamin mit geringerer Verfärbung durch Zusatz von phosphoriger oder unterphosphoriger Säure oder deren Derivate vor oder während oder direkt nach der stufenweisen Umsetzung von Ethylenoxid mit Ammoniak und anschließende Isolierung durch Destillation erhalten werden.

WO-A-00/32553 (BASF AG) betrifft ein Verfahren zur Reinigung von TEOA, hergestellt durch Umsetzung von wässrigem Ammoniak mit Ethylenoxid in flüssiger Phase unter Druck und bei erhöhter Temperatur, indem man vom Umsetzungsprodukt überschüssiges Ammoniak, Wasser und Monoethanolamin abtrennt, das so erhaltene Rohprodukt mit Ethylenoxid umsetzt und anschließend in Gegenwart von phosphoriger oder unterphosphoriger Säure oder deren Verbindungen rektifiziert.

EP-A-1 132 371 (BASF AG) betrifft ein Verfahren zur Herstellung von Alkanolaminen mit verbesserter Farbqualität, wobei man das Alkanolamin mit einer wirksamen Menge von phosphoriger oder unterphosphoriger Säure oder deren Verbindungen zunächst bei erhöhter Temperatur über einen Zeitraum von mindestens 5 Min. behandelt (Schritt a) und anschließend in Gegenwart einer wirksamen Menge einer dieser Phosphorverbindungen destilliert (Schritt b).

Der vorliegenden Erfindung lag die Aufgabe zugrunde, ein gegenüber dem Stand der Technik verbessertes Verfahren zur Herstellung von Triethanolamin mit hoher Farbqualität bereitzustellen. Das Verfahren soll die Verfärbung von TEOA, z. B. gemessen als APHA-Farbzahl, vermindern und die Farbstabilität verbessern (unerwünschte Zunahme der Farbzahl über die Lagerzeit). Insbesondere sollte das Verfahren gegenüber EP-A-4015, WO-A-00/32553 und EP-A-1 132 371 höhere Ausbeuten an TEOA liefern.

Demgemäß wurde ein Verfahren zur Herstellung von Triethanolamin gefunden, welches dadurch gekennzeichnet ist, dass man dem Triethanolamin phosphorige und/oder unterphosphorige Säure und eine basische Verbindung, ausgewählt aus Alkalimetallhydroxid, Erdalkalimetallhydroxid und [R¹R²R³(2-hydroxyethyl)ammonium]hydroxid, wobei R¹, R² und R³ unabhängig voneinander C₁₋₃₀-Alkyl oder C₂₋₁₀-Hydroxyalkyl bedeuten, zusetzt und im Fall von Alkalimetallhydroxid als basischer Verbindung das Mol-Verhältnis Säure(n) : Hydroxid im Bereich von 1 : 0,1 bis 1 : 1 liegt und im Fall von Erdalkalimetallhydroxid als basischer Verbindung das Mol-Verhältnis Säure(n) : Hydroxid im Bereich von 1 : 0,05 bis 1 : 0,5 liegt.

Weiterhin wurde Triethanolamin enthaltend phosphorige und/oder unterphosphorige Säure und R¹R²R³(2-hydroxyethyl)ammonium]hydroxid, wobei R¹, R² und R³ unabhängig voneinander C₁₋₃₀-Alkyl oder C₂₋₁₀-Hydroxyalkyl bedeuten, gefunden.

Weiterhin wurde Triethanolamin enthaltend phosphorige und/oder unterphosphorige Säure und ein Alkalimetallhydroxid oder Erdalkalimetallhydroxid, wobei im Fall von Alkalimetallhydroxid das Mol-Verhältnis Säure(n) : Hydroxid im Bereich von 1 : 0,1 bis 1 : 1 liegt und im Fall von Erdalkalimetallhydroxid das Mol-Verhältnis Säure(n) : Hydroxid im Bereich von 1 : 0,05 bis 1 : 0,5 liegt, gefunden.

Bevorzugte Mol-Verhältnisse Säure(n) : Hydroxid im Triethanolamin sind der Beschreibung weiter unten zu entnehmen.

Erfindungsgemäß wurde erkannt, dass unter Beibehaltung oder sogar Verbesserung der Farbqualität gegenüber der alleinigen Anwendung von H₃PO₃ oder H₃PO₂, durch die zusätzliche basische Verbindung die Bildung von Nebenprodukten im TEOA erheblich vermindert wird (Pufferwirkung der Base). Gleichzeitig wird die TEOA-Destillationsausbeute gesteigert. Die Nebenproduktbildung beruht vermutlich auf der sauren Wirkung der Phosphorverbindungen.

Das im erfindungsgemäßen Verfahren eingesetzte Triethanolamin kann nach bekannten Verfahren, insbesondere durch Umsetzung von Ammoniak mit Ethylenoxid (z. B. gemäß EP-A-673 920 oder WO-A-00132553) erhalten werden.

Die Reinheit des im erfindungsgemäßen Verfahren eingesetzten Triethanolamins beträgt bevorzugt größer 70 Gew.-%, insbesondere größer 80 Gew.-%. Neben destilliertem oder undestilliertem rohen Triethanolamin, das auch direkt in roher Form einer Anlage zur Herstellung des Alkanolamins aus den entsprechenden Vorstufen entnommen werden kann, kann auch destilliertes TEOA mit einer Reinheit von größer 90 Gew.-%, z.B. größer 95 Gew.-%, besonders ≥ 97 Gew.-%, insbesondere ≥ 98 Gew.-%, ganz besonders ≥ 99 Gew.-%, eingesetzt werden.

Es können auch Mischungen von Triethanolamin mit anderen Alkanolaminen, wie z.B. Monoethanolamin (MEA), Diethanolamin (DEA), Aminodiglykol (ADG, H₂NCH₂CH₂OCH₂CH₂OH), O,N,N-Tris-(2-hydroxyethyl)-ethanolamin, N-(2-Aminoethyl)-ethanolamin (AEEA), N-(2-Hydroxyethyl)-piperazin, N-(2-Hydroxyethyl)-morpholin, N,N'-Bis-(2-Hydroxyethyl)-piperazin, Monoisopropanolamin, Diisopropanolamin, Triisopropanolamin und 1,3-Propanolamin, oder Lösungen von Triethanolamin in einem inerten Lösungsmittel, wie z. B. Alkohole (Methanol, Ethanol, iso-Propanol, n-Propanol, n-Butanol, 2-Ethylhexanol), Ether (Tetrahydrofuran, 1,4-Dioxan), Kohlenwasserstoffe (Benzol, Pentan, Petrolether, Toluol, Xylol, Hexan, Heptan, Mihagol) und Wasser oder Mischungen hiervon, eingesetzt werden.

Die APHA-Farbzahl des eingesetzten Triethanolamins beträgt bevorzugt ≤ 100, insbesondere ≤ 50, ganz besonders ≤ 20.

Das erfindungsgemäße Verfahren lässt sich wie folgt ausführen:

Das in seiner Farbqualität zu verbessernde Triethanolamin in flüssiger Phase, optional in Gegenwart eines inerten Lösungsmittels, wird in einem geeigneten Behälter, z. B. Rührbehälter, der mit einem Rückflusskühler ausgerüstet sein kann, mit einer wirksamen Menge von phosphoriger Säure (H₃PO₃) und/oder unterphosphoriger Säure (H₃PO₂) und mit einer basischen Verbindung, ausgewählt aus Alkalimetallhydroxid, Erdalkalimetallhydroxid und [R¹R²R³(2-hydroxyethyl)ammonium]hydroxid, wobei R¹, R² und R³ die genannten Bedeutungen aufweisen, vorteilhaft unter Rühren oder Umpumpen, versetzt.

Die Mischung wird über einen Zeitraum von bevorzugt mindestens 5 Min., insbesondere mindestens 10 Min. (beispielsweise 10 Min. bis 50 Stunden, insbesondere 10 Min. bis 24 Stunden), ganz besonders mindestens 15 Min. (beispielsweise 15 Min. bis 6 Stunden), besonders bevorzugt mindestens 30 Min. (beispielsweise 30 Min. bis 4 Stunden oder 40 Min. bis 3 Stunden oder 60 Min. bis 2 Stunden), auf eine Temperatur im Bereich von 40 bis 250 °C, insbesondere 100 bis 240 °C, ganz besonders 120 bis 230 °C, besonders bevorzugt 150 bis 220 °C, erwärmt.

Die phosphorige Säure und/oder unterphosphorige Säure kann im erfindungsgemäßen Verfahren in monomerer oder auch in polymerer Form, in wasserhaltiger Form (Hydrate oder wässrige Lösung oder wässrige Suspension) oder als Anlagerungsverbindung (z. B. auf einem anorganischen oder organischen Träger wie SiO₂, Al₂O₃, TiO₂, ZrO₂) eingesetzt werden.

Die Menge an zugesetzter/en Säure/n beträgt in der Regel mindestens 0,01 Gew.-%, bevorzugt 0,02 bis 2 Gew.-%, besonders bevorzugt 0,03 bis 1,0 Gew.%, ganz besonders bevorzugt 0,5 bis 0,9 Gew.-%, bezogen auf die eingesetzte Menge an Triethanolamin (berechnet reine Ware); die Wirkung tritt jedoch auch bei größeren Mengen ein. Werden phosphorige Säure und unterphosphorige Säure zusammen eingesetzt, beziehen sich die obigen Mengenangaben auf beide Säuren zusammen.

Als basische Verbindung kann im erfindungsgemäßen Verfahren ein Alkalimetallhydroxid, mit Alkalimetall = Li, Na, K, Rb oder Cs, bevorzugt Na oder K, ein Erdalkalimetallhydroxid, mit Erdalkalimetall = Be, Mg, Ca, Sr, Ba, oder bevorzugt ein Ammoniumhydroxid der Formel [R¹R²R³(2-hydroxyethyl)ammonium]hydroxid, also 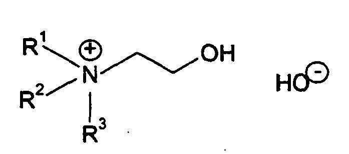 eingesetzt werden.

Die Reste R¹, R² und R³ haben unabhängig voneinander die folgende Bedeutung:

Unverzweigtes oder verzweigtes C₁₋₃₀-Alkyl, darunter C₈₋₂₂-Alkyl, bevorzugt C₁₋₂₀-Alkyl, insbesondere C₁₋₁₄-Alkyl, darunter C₁₋₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl,-1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Undecyl, n-Dodecyl, n-Tridecyl, 2-n-Butyl-n-nonyl, 3-n-Butyl-n-nonyl, n-Tetradecyl, n-Pentadecyl, n-Hexadecyl, n-Octadecyl,

C₂₋₁₀-Hydroxyalkyl, bevorzugt C₂₋₈-Hydroxyalkyl, besonders bevorzugt C₂₋₄-Hydroxyalkyl, wie 2-Hydroxyethyl, 2-Hydroxy-n-propyl, 3-Hydroxy-n-propyl und 1-(Hydroxymethyl)ethyl, besonders bevorzugt 2-Hydroxyethyl.

Diese 2-(Hydroxyethyl)ammoniumhydroxide sind dem Fachmann nach bekannten Verfahren zugänglich. Sie sind insbesondere durch die Umsetzung des entsprechenden tertiären Amins R¹R²R³N (z.B. Et₃N, Fettamin, TEOA) mit einem Moläquivalent Ethylenoxid und Wasser zugänglich.
Vergl. für [Tetrakis(2-hydroxyethyl)ammonium]hydroxid z.B.: A.R. Doumaux et al. J. Org. Chem. 1973, 38 (20), Seiten 3630-3632, und DE-A-22 17 494 und DE-A-21 21 325 (beide BASF AG).

Ein Vorteil des Ammoniumhydroxids besteht darin, dass sich das quaternäre Ammoniumsalz vollständig in der Triethanolamin-Mischung löst und die H₃PO₃ und/oder H₃PO₂ zumindest teilweise neutralisiert (Pufferwirkung).

Besonders bevorzugte Ammoniumhydroxide sind [Tetrakis(2-hydroxyethyl)ammonium]-hydroxid und [(C₁₋₄-Alkyl)₃(2-hydroxyethyl)ammonium]hydroxid, wie z.B. [Triethyl(2-hydroxyethyl)ammonium]hydroxid.

Ein weiterer Vorteil des besonders bevorzugten [Tetrakis(2-hydroxyethyl)ammonium]-hydroxids ergibt sich daraus, dass sich die Base unter den Bedingungen der Triethanolamin-Destillation teilweise oder vollständig zu Wasser und dem Wertprodukt Triethanolamin zersetzt und daher aus dem Wertprodukt nicht abgetrennt zu werden braucht. Es treten keine Probleme durch Salzbildung auf.

Ein weiterer Vorteil eines bevorzugten, auf einem Fettamin ((C₈₋₃₀)₃N) basierenden (2-hydroxyethyl)ammoniumhydroxids besteht darin, dass sich die Base unter den Bedingungen der Triethanolamin-Destillation teilweise oder vollständig zu Wasser und dem Fettamin zersetzt und das Fettamin als Hochsieder leicht über den Destillationssumpf vom reinen TEOA abgetrennt werden kann.

Bevorzugt beträgt das Mol-Verhältnis von eingesetzter/en Säure/n zu eingesetztem Ammoniumhydroxid 1 : 1 bis 100 : 1, besonders 1,1 : 1 bis 10 : 1, insbesondere 1,2 : 1 bis 8 : 1, ganz besonders 1,3 : 1 bis 6 : 1.

Im Fall von Alkalimetallhydroxid als basischer Verbindung liegt das Mol-Verhältnis Säure(n) : Hydroxid bevorzugt im Bereich von 1 : 0,2 bis 1 : 0,9, insbesondere 1 : 0,3 bis 1 : 0,8, ganz besonders 1 : 0,4 bis 1 : 0,7, z.B. 1 : 0,5 bis 1 : 0,6.

Im Fall von Erdalkalimetallhydroxid als basischer Verbindung liegt das Mol-Verhältnis Säure(n) : Hydroxid bevorzugt im Bereich von 1 : 0,1 bis 1 : 0,45, insbesondere 1 : 0,15 bis 1 : 0,4, ganz besonders 1 : 0,2 bis 1 : 0,35.

Werden phosphorige Säure und unterphosphorige Säure zusammen eingesetzt, beziehen sich die obigen Mol-Verhältnis-Angaben auf beide Säuren zusammen.

Beispielsweise werden dem TEOA 1000 ppm H₃PO₃ und zwischen 320 und 2573 ppm der basischen Verbindung [Tetrakis(2-hydroxyethyl)ammonium]hydroxid zugegeben.

Man kann in das TEOA zuerst die basische Verbindung und danach die Säure/n geben.
In einer bevorzugten Verfahrensweise wird/werden zuerst die Säure/n in das TEOA gegeben und anschließend die basische Verbindung.
In einer anderen bevorzugten Verfahrensweise wird zuerst eine Mischung der Säure(n) mit der basischen Verbindung hergestellt und diese Mischung dann dem TEOA zugegeben.

Für die bessere Handhabbarkeit kann es dabei von Vorteil sein, die wirksame Menge von phosphoriger Säure und/oder unterphosphoriger Säure in einem geeigneten inerten Verdünnungs- oder Lösungsmittel, wie z. B. Wasser, Alkohole (Methanol, Ethanol, iso-Propanol, n-Propanol, n-Butanol, 2-Butanol), Ether (Tetrahydrofuran, 1,4-Dioxan) oder einem Alkanolamin (z. B. einem Ethanolamin, wie Monoethanolamin, Diethanolamin, N-(2-Aminoethyl)-ethanolamin, insbesondere Triethanolamin), in Form einer Lösung oder einer Suspension zuzudosieren.

Die basische Verbindung kann vorteilhaft als Lösung oder Suspension in Wasser eingesetzt werden, z.B. als 30 bis 80 Gew.%ige, insbesondere 40 bis 60 Gew.-%ige, Lösung oder Suspension.
[Tetrakis(2-hydroxyethyl)ammonium]hydroxid ist als 50 Gew.-%ige wässrige Lösung kommerziell erhältlich und vorteilhaft einsetzbar.

Die erforderliche Behandlungsdauer des Triethanolamins mit dem Zusatz aus Säure und basischer Verbindung ergibt sich unter anderem aus dem Grad der Verfärbung des eingesetzten Triethanolamins und dem Ausmaß der gewünschten Entfärbung und/oder Farbstabilität des TEOAs. Sie ist in der Regel bei gegebener Temperatur umso größer, je höher der Grad der Verfärbung des im erfindungsgemäßen Verfahren eingesetzten Triethanolamins ist und je höher die Anforderungen an die farbliche Qualität des Verfahrensproduktes sind.
Die Temperatur darf jedoch nicht zu hoch, d. h. in der Regel nicht höher als 250 °C, gewählt sein, da ansonsten eine säureinduzierte Degradation des Triethanolamins eintreten kann, die die Farbqualität des schließlich erhaltenen TEOAs negativ beeinflusst. Die für das jeweilig eingesetzte Triethanolamin günstigsten Temperaturen und Behandlungsdauern sind in einfachen Vorversuchen leicht zu ermitteln.

Bei dieser Behandlung des Triethanolamins mit der Säure und der basischen Verbindung ist es von Vorteil, wenn die Mischung während der gesamten Behandlungsdauer oder zeitweise weiter durchmischt (z. B. gerührt oder im Kreislauf umgepumpt) wird.

Weiterhin ist es von Vorteil, wenn die Behandlung des Triethanolamins unter einer Schutzgasatmosphäre (z. B. N₂ oder Ar), also in Abwesenheit von O₂, durchgeführt wird.

Die Behandlung des Alkanolamins mit der Säure und der basischen Verbindung kann auch kontinuierlich in geeigneten Behältern, z. B. in einem Rohrreaktor oder in einer Rührbehälterkaskade, durchgeführt werden.

Die Behandlung des Triethanolamins mit der Säure und der basischen Verbindung kann vorteilhaft im Sumpfbehälter einer Destillationskolonne oder in einem Destillationsvorlagegefäß vor und/oder während der Destillation des Triethanolamins durchgeführt werden.

In einer besonderen Ausgestaltung wird während der Behandlung des Triethanolamins mit der Säure und der basischen Verbindung ein Inertgas (z. B. N₂ oder Ar) als Strippstrom durch das Triethanolamin geleitet, um entstehende Leichtsieder, die sich negativ auf die Farbqualität auswirken können, wie z. B. Acetaldehyd oder dessen Folgeprodukte, aus dem Gemisch zu entfernen.

In einer anderen besonderen Ausgestaltung wird das zu behandelnde Triethanolamin im Kreislauf flüssig über einen Wärmetauscher geführt und werden entstehende Leichtsieder, die sich negativ auf die Farbqualität auswirken können, wie z. B. Acetaldehyd, dabei abgeführt.
Bei dem Wärmetauscher kann es sich hierbei um einen offenen Wärmetauscher, wie z. B. einen Fallfilm- oder Wischblattverdampfer, oder einen geschlossenen Wärmetauscher, wie z. B. einen Platten- oder Rohrbündelwärmetauscher, handeln.

Je nach den gewählten Reaktionsbedingungen kann es notwendig sein, die Behandlung des Triethanolamins mit der Säure und der basischen Verbindung bei einem Überdruck (z. B. 0,1 bis 50 bar) durchzuführen, um das unerwünschte Entweichen einer oder mehrerer Komponenten aus der Mischung zu vermeiden.

Die Destillation oder Rektifikation des Triethanolamins zur Abtrennung der zugesetzten Verbindungen erfolgt diskontinuierlich oder kontinuierlich bei einem Druck von in der Regel kleiner 100 mbar (100 hPa), beispielsweise bei ca. 10 bis 50 mbar oder 1 bis 20 mbar, bevorzugt bei 0,5 bis 5 mbar, und bei Sumpftemperaturen von in der Regel 100 bis 250 °C, wobei bei der kontinuierlichen Fahrweise in einer besonderen Ausgestaltung gegebenenfalls vorhandene Leichtsiederanteile über Kopf abgezogen werden und man das TEOA im Seitenabzug erhält.

Der die zugesetzten Verbindungen und/oder deren Umsetzungsprodukte enthaltende Rückstand der Destillation oder Rektifikation kann in einer besonderen Ausführungsform vollständig oder teilweise in das Destillationsverfahren zurückgeführt werden.

Das erfindungsgemäße Verfahren liefert ein in der Farbqualität verbessertes Triethanolamin, das direkt nach seinem Erhalt eine APHA-Farbzahl im Bereich von 0 bis 30, insbesondere von 0 bis 20, ganz besonders von 0 bis 10, z.B. 1 bis 6, aufweist.

Alle APHA-Angaben in diesem Dokument erfolgen nach DIN ISO 6271(= Hazen). Alle ppm-Angaben in diesem Dokument beziehen sich auf das Gewicht (Gew.-ppm).

### Beispiele

Die Versuche wurden in einer Laborapparatur, bestehend aus einem 4 Liter Dreihalskolben mit Rührer, Thermometer und Gaszuleitung durchgeführt. Zu einer Mischung aus 21 Gew.-% Diethanolamin und 79 Gew.% Triethanolamin wurden 1000 ppm H₃PO₃ gegeben und jeweils wahlweise unterschiedliche Mengen einer Base.

Im Vakuum wurden aus dem Kolben bei etwa 190-195°C Sumpftemperatur nacheinander Diethanolamin und Triethanolamin in einem Zeitraum im Bereich von 1 bis 8 h über eine Vigreux-Kolonne abdestilliert und Fraktionen an Triethanolamin mit einem Gehalt von mindestens 99,4 % (GC-FL%) erhalten.

Von diesen Qualitäten Triethanolamin wurden Farbzahlmessungen (nach Hazen) durchgeführt und nachfolgend tabellarisch dokumentiert. Die Ausbeute-Verluste durch Nebenreaktionen wurden durch das Auswiegen der erhaltenen Fraktionen an Diethanolamin und Triethanolamin (TEOA) bestimmt und beziehen sich auf die Bildung von schwersiedenden Verbindungen, welche nach der Destillation im Sumpf verblieben sind.

**Tabelle 1:**

| Art, Menge des Zusatzes (ppm) | Base/H₃PO₃ (Mol-Verhältnis) | Gehalt TEOA (GC-FI.%) | Farbzahl (Hazen) | Menge Sumpfrückstand (Gew.-%) |
|---|---|---|---|---|
| | | | | |
| H₃PO₃, 1000 | 0 | 99,6 | 5 | 5,0 |
| H₃PO₃, 1000 / Base, 320 | 1/8 | 99,5 | 2 | 2,2 |
| H₃PO₃, 1000 / Base, 645 | ¼ | 99,7 | 5 | 4,2 |
| H₃PO₃, 1000 / Base, 1290 | ½ | 99,5 | 3 | 1,1 |

| | | | | |
|---|---|---|---|---|
| Base = [Tetrakis(2-hydroxyethyl)ammonium]hydroxid | | | | |

Die zusätzliche Gabe des Ammoniumhydroxids zur phosphorigen Säure bewirkt eine Erhöhung der Destillationsausbeute an Triethanolamin ohne negativem Einfluss auf die Farbzahl, teilweise sogar mit positivem Einfluss auf die Farbzahl (im Sinne einer Verringerung der Farbzahl).

**Tabelle 2:**

| Art, Menge des Zusatzes (ppm) | NaOH/H₃PO₃ (Mol-Verhältnis) | Gehalt TEOA (GC-Fl.%) | Farbzahl (Hazen) | Menge Sumpfrückstand (Gew.-%) |
|---|---|---|---|---|
| | | | | |
| H₃PO₃ , 1000 / NaOH, 490 | 1,0 | 99,8 | 3 | 2,0 |

## Patentansprüche

1. Verfahren zur Herstellung von Triethanolamin, **dadurch gekennzeichnet, dass** man dem Triethanolamin phosphorige und/oder unterphosphorige Säure und eine basische Verbindung, ausgewählt aus Alkalimetallhydroxid, Erdalkalimetallhydroxid und [R¹R²R³(2-hydroxyethyl)ammonium]hydroxid, wobei R¹, R² und R³ unabhängig voneinander C₁₋₃₀-Alkyl oder C₂₋₁₀-Hydroxyalkyl bedeuten, zusetzt und im Fall von Alkalimetallhydroxid als basischer Verbindung das Mol-Verhältnis Säure(n) : Hydroxid im Bereich von 1 : 0,1 bis 1 : 1 liegt und im Fall von Erdalkalimetallhydroxid als basischer Verbindung das Mol-Verhältnis Säure(n) : Hydroxid im Bereich von 1 : 0,05 bis 1 : 0,5 liegt.

2. Verfahren nach Anspruch 1, **dadurch gekennzeichnet, dass** man dem Triethanolamin die phosphorige und/oder unterphosphorige Säure und die basische Verbindung vor und/oder während der Destillation des Triethanolamins zusetzt.

3. Verfahren nach einem der Ansprüche 1 oder 2, **dadurch gekennzeichnet, dass** man das Triethanolamin mit dem Zusatz an Säure(n) und basischer Verbindung über einen Zeitraum von mindestens 5 Min. behandelt.

4. Verfahren nach einem der Ansprüche 1 oder 2, **dadurch gekennzeichnet, dass** man das Triethanolamin mit dem Zusatz an Säure(n) und basischer Verbindung über einen Zeitraum im Bereich von 10 Min. bis 50 Stunden behandelt.

5. Verfahren nach einem der beiden vorhergehenden Ansprüche, **dadurch gekennzeichnet, dass** man die Behandlung des Triethanolamins mit dem Zusatz bei einer Temperatur im Bereich von 40 bis 250 °C durchführt.

6. Verfahren nach einem der vorhergehenden Ansprüche, **dadurch gekennzeichnet, dass** man dem Triethanolamin 0,01 bis 2 Gew.-% (bezogen auf das reine Triethanolamin) phosphorige und/oder unterphosphorige Säure zusetzt.

7. Verfahren nach einem der vorhergehenden Ansprüche, **dadurch gekennzeichnet, dass** man dem Triethanolamin phosphorige und/oder unterphosphorige Säure und R¹R²R³(2-hydroxyethyl)ammonium]hydroxid in einem Mol-Verhältnis Säure(n) : Hydroxid im Bereich von 1 : 1 bis 100: 1 zusetzt.

8. Verfahren nach einem der Ansprüche 1 bis 6, **dadurch gekennzeichnet, dass** man dem Triethanolamin phosphorige und/oder unterphosphorige Säure und R¹R²R³(2-hydroxyethyl)ammonium]hydroxid in einem Mol-Verhältnis Säure(n) : Hydroxid im Bereich von 1,1 : 1 bis 10 : 1 zusetzt.

9. Verfahren nach einem der vorhergehenden Ansprüche, **dadurch gekennzeichnet, dass** es sich bei dem Ammoniumhydroxid um [Tetrakis(2-hydroxyethyl)ammonium]hydroxid oder [(C₁₋₄-Alkyl)₃(2-hydroxyethyl)ammonium]hydroxid handelt.

10. Verfahren nach einem der Ansprüche 1 bis 8, **dadurch gekennzeichnet, dass** es sich bei dem Ammoniumhydroxid um [Triethyl(2-hydroxyethyl)ammonium]-hydroxid handelt.

11. Triethanolamin enthaltend phosphorige und/oder unterphosphorige Säure und R¹R²R³(2-hydroxyethyl)ammonium]hydroxid, wobei R¹, R² und R³ unabhängig voneinander C₁₋₃₀-Alkyl oder C₂₋₁₀-Hydroxyalkyl bedeuten.

12. Triethanolamin nach dem vorhergehenden Anspruch enthaltend 0,01 bis 2 Gew.-% (bezogen auf das reine Triethanolamin) phosphorige und/oder unterphosphorige Säure.

13. Triethanolamin nach einem der beiden vorhergehenden Ansprüche enthaltend phosphorige und/oder unterphosphorige Säure und R¹R²R³(2-hydroxyethyl)-ammonium]hydroxid in einem Mol-Verhältnis Säure(n) : Hydroxid im Bereich von 1 : 1 bis 100: 1.

14. Triethanolamin nach einem der drei vorhergehenden Ansprüche enthaltend [Tetrakis(2-hydroxyethyl)ammonium]hydroxid.

15. Triethanolamin nach einem der Ansprüche 11 bis 13 enthaltend [(C₁₋₄-Alkyl)₃(2-hydroxyethyl)ammonium]hydroxid.

16. Triethanolamin enthaltend phosphorige und/oder unterphosphorige Säure und ein Alkalimetallhydroxid oder Erdalkalimetallhydroxid, wobei im Fall von Alkalimetallhydroxid das Mol-Verhältnis Säure(n) : Hydroxid im Bereich von 1 : 0,1 bis 1 : 1 liegt und im Fall von Erdalkalimetallhydroxid das Mol-Verhältnis Säure(n) : Hydroxid im Bereich von 1 : 0,05 bis 1 : 0,5 liegt.

17. Triethanolamin nach dem vorhergehenden Anspruch enthaltend 0,01 bis 2 Gew.-% (bezogen auf das reine Triethanolamin) phosphorige und/oder unterphosphorige Säure.

18. Triethanolamin nach einem der beiden vorhergehenden Ansprüche enthaltend Natriumhydroxid.

## Claims

1. A method of producing triethanolamine wherein phosphorous and/or hypophosphorous acid and a basic compound chosen from alkali metal hydroxide, alkaline earth metal hydroxide and [R¹R²R³(2-hydroxyethyl)ammonium] hydroxide, where R¹, R² and R³, independently of one another, are C₁₋₃₀-alkyl or C₂₋₁₀-hydroxyalkyl, are added to the triethanolamine and in the case of alkali metal hydroxide as basic compound the molar ratio of acid(s):hydroxide is in the range from 1:0.1 to 1:1 and in the case of alkaline earth metal hydroxide as basic compound the molar ratio of acid(s):hydroxide is in the range from 1:0.05 to 1:0.5.

2. The method according to claim 1, wherein the phosphorous and/or hypophosphorous acid and the basic compound are added to the triethanolamine before and/or during distillation of the triethanolamine.

3. The method according to either claim 1 or 2, wherein the triethanolamine is treated with the addition of acid(s) and basic compound over a period of at least 5 min.

4. The method according to either claim 1 or 2, wherein the triethanolamine is treated with the addition of acid(s) and basic compound over a period in the range from 10 min to 50 hours.

5. The method according to one of the two preceding claims, wherein the treatment of the triethanolamine with the addition is carried out at a temperature in the range from 40 to 250°C.

6. The method according to one of the preceding claims, wherein 0.01 to 2% by weight (based on the pure triethanolamine) of phosphorous and/or hypophosphorous acid are added to the triethanolamine.

7. The method according to one of the preceding claims, wherein phosphorous and/or hypophosphorous acid and R¹R²R³(2-hydroxyethyl)ammonium] hydroxide is added in a molar ratio of acid(s):hydroxide in the range from 1:1 to 100:1 to the triethanolamine.

8. The method according to one of claims 1 to 6, wherein phosphorous and/or hypophosphorous acid and R¹R²R³(2-hydroxyethyl)ammonium] hydroxide is added in a molar ratio of acid(s): hydroxide in the range from 1.1:1 to 10:1 to the triethanolamine.

9. The method according to one of the preceding claims, wherein the ammonium hydroxide is [tetrakis(2-hydroxyethyl)ammonium] hydroxide or [(C₁₋₄-alkyl)₃(2-hydroxyethyl)ammonium] hydroxide.

10. The method according to one of claims 1 to 8, wherein the ammonium hydroxide is [triethyl(2-hydroxyethyl)ammonium] hydroxide.

11. A triethanolamine comprising phosphorous and/or hypophosphorous acid and R¹R²R³(2-hydroxyethyl)ammonium] hydroxide, where R¹, R² and R³, independently of one another, are C₁₋₃₀-alkyl or C₂₋₁₀-hydroxyalkyl.

12. The triethanolamine according to the preceding claim comprising 0.01 to 2% by weight (based on the pure triethanolamine) of phosphorous and/or hypophosphorous acid.

13. The triethanolamine according to one of the two preceding claims comprising phosphorous and/or hypophosphorous acid and R¹R²R³(2-hydroxyethyl)-ammonium] hydroxide in a molar ratio of acid(s):hydroxide in the range from 1:1 to 100:1.

14. The triethanolamine according to one of the three preceding claims comprising [tetrakis(2-hydroxyethyl)ammonium] hydroxide.

15. The triethanolamine according to one of claims 11 to 13 comprising [(C₁₋₄-alkyl)₃(2-hydroxyethyl)-ammonium] hydroxide.

16. A triethanolamine comprising phosphorous and/or hypophosphorous acid and an alkali metal hydroxide or alkaline earth metal hydroxide, where in the case of alkali metal hydroxide the molar ratio of acid(s):hydroxide is in the range from 1:0.1 to 1:1 and in the case of alkaline earth metal hydroxide the molar ratio of acid(s):hydroxide is in the range from 1:0.05 to 1:0.5.

17. The triethanolamine according to the preceding claim comprising 0.01 to 2% by weight (based on the pure triethanolamine) of phosphorous and/or hypophosphorous acid.

18. The triethanolamine according to one of the two preceding claims comprising sodium hydroxide.

## Revendications

1. Procédé de préparation de triéthanolamine, **caractérisé en ce que** l'on ajoute à la triéthanolamine de l'acide phosphoreux et/ou hypophosphoreux et un composé basique choisi parmi un hydroxyde de métal alcalin, un hydroxyde de métal alcalino-terreux et un hydroxyde de [R¹R²R³(2-hydroxyéthyl)ammonium], où R¹, R² et R³ représentent indépendamment les uns des autres des radicaux alkyle en C₁ à C₃₀ ou hydroxyalkyle en C₂ à C₁₀, et, dans le cas d'un hydroxyde de métal alcalin en tant que composé basique, la proportion molaire acide(s) : hydroxyde est de l'ordre de 1:0,1 à 1:1 et, dans le cas d'un hydroxyde de métal alcalino-terreux en tant que composé basique, la proportion molaire acide(s) : hydroxyde est de l'ordre de 1:0,05 à 1:0,5.

2. Procédé suivant la revendication 1, **caractérisé en ce que** l'on ajoute à la triéthanolamine l'acide phosphoreux et/ou hypophosphoreux et le composé basique avant et/ou pendant la distillation de la triéthanolamine.

3. Procédé suivant l'une quelconque des revendications 1 ou 2, **caractérisé en ce que** l'on traite la triéthanolamine par l'addition d'acide(s) et de composé basique en l'espace d'au moins 5 minutes.

4. Procédé suivant l'une quelconque des revendications 1 ou 2, **caractérisé en ce que** l'on traite la triéthanolamine par l'addition d'acide(s) et de composé basique en l'espace de 10 à 50 heures.

5. Procédé suivant l'une quelconque des deux revendications qui précèdent, **caractérisé en ce que** l'on entreprend le traitement de la triéthanolamine par l'addition à une température de l'ordre de 40 à 250°C.

6. Procédé suivant l'une quelconque des revendications qui précèdent, **caractérisé en ce que** l'on ajoute à la triéthanolamine de 0,01 à 2 % en poids (par rapport à la triéthanolamine pure) d'acide phosphoreux et/ou hypophosphoreux.

7. Procédé suivant l'une quelconque des revendications qui précèdent, **caractérisé en ce que** l'on ajoute à la triéthanolamine de l'acide phosphoreux et/ou hypophosphoreux et de l'hydroxyde de [R¹R²R³(2-hydroxyéthyl)ammonium] en une proportion molaire acide(s) : hydroxyde de l'ordre de 1:1 à 100:1.

8. Procédé suivant l'une quelconque des revendications 1 à 6, **caractérisé en ce que** l'on ajoute à la triéthanolamine de l'acide phosphoreux et/ou hypophosphoreux et de l'hydroxyde de [R¹R²R³(2-hydroxyéthyl)ammonium] en une proportion molaire acide(s) : hydroxyde de l'ordre de 1,1:1 à 10:1.

9. Procédé suivant l'une quelconque des revendications qui précèdent, **caractérisé en ce que** l'hydroxyde d'ammonium est de l'hydroxyde de [tétrakis(2-hydroxyéthyl) ammonium] ou de l'hydroxyde de [((C₁-C₄) alky)₃(2-hydroxyéthyl) ammonium].

10. Procédé suivant l'une quelconque des revendications 1 à 8, **caractérisé en ce que** l'hydroxyde d'ammonium est de l'hydroxyde de [triéthyl(2-hydroxyéthyl)ammonium].

11. Triéthanolamine contenant de l'acide phosphoreux et/ou hypophosphoreux et de l'hydroxyde de [R¹R²R³(2-hydroxyéthyl)ammonium], où R¹, R² et R³ représentent indépendamment les uns des autres des radicaux alkyle en C₁ à C₃₀ ou hydroxyalkyle en C₂ à C₁₀.

12. Triéthanolamine suivant la revendication qui précède, contenant de 0,01 à 2 % en poids (par rapport à la triéthanolamine pure) d'acide phosphoreux et/ou hypophosphoreux.

13. Triéthanolamine suivant l'une quelconque des deux revendications qui précèdent, contenant de l'acide phosphoreux et/ou hypophosphoreux et de l'hydroxyde de [R¹R²R³(2-hydroxyéthyl)ammonium] en une proportion molaire acide(s) : hydroxyde de l'ordre de 1:1 à 100:1.

14. Triéthanolamine suivant l'une quelconque des trois revendications qui précèdent, contenant de l'hydroxyde de [tétrakis(2-hydroxyéthyl)ammonium].

15. Triéthanolamine suivant l'une quelconque des revendications 11 à 13, contenant de l'hydroxyde de [((C₁-C₄)alkyl)₃(2-hydroxyéthyl)ammonium].

16. Triéthanolamine contenant de l'acide phosphoreux et/ou hypophosphoreux et un hydroxyde de métal alcalin ou un hydroxyde de métal alcalino-terreux, où dans le cas d'un hydroxyde de métal alcalin, la proportion molaire acide(s) : hydroxyde est de l'ordre de 1:0,1 à 1:1 et, dans le cas d'un hydroxyde de métal alcalino-terreux, la proportion molaire acide(s) : hydroxyde est de l'ordre de 1:0,05 à 1:0,5.

17. Triéthanolamine suivant la revendication qui précède, contenant de 0,01 à 2 % en poids (par rapport à la triéthanolamine pure) d'acide phosphoreux et/ou hypophosphoreux.

18. Triéthanolamine suivant l'une quelconque des deux revendications qui précèdent, contenant de l'hydroxyde de sodium.
